# EUROPEAN PATENT SPECIFICATION

(11) **EP 2 424 437 B1**
(45) Date of publication and mention of the grant of the patent: **16.10.2013**
(21) Application number: 10713255.7
(22) Date of filing: 12.03.2010
(51) Int. Cl.: A61B 6/03, G01T 1/20

(54) **IMAGING APPARATUS AND IMAGING SYSTEM, AND CONTROL METHOD AND PROGRAM FOR THE SAME**
BILDGEBUNGSGERÄT UND BILDGEBUNGSSYSTEM UND KONTROLLVERFAHREN UND PROGRAMM DAFÜR
APPAREIL D'IMAGERIE ET SYSTEME D'IMAGERIE, ET PROCEDE DE COMMANDE ET PROGRAMME POUR CELUI-CI

(30) Priority: 01.05.2009 JP 2009112052
(43) Date of publication of application: 07.03.2012
(73) Proprietor: Canon Kabushiki Kaisha, Tokyo 146-8501 (JP)
(72) Inventor: KAMESHIMA, Toshio, Tokyo 146-8501 (JP); ENDO, Tadao, Tokyo 146-8501 (JP); AKIYAMA, Masayoshi, Tokyo 146-8501 (JP); YAGI, Tomoyuki, Tokyo 146-8501 (JP); TAKENAKA, Katsuro, Tokyo 146-8501 (JP); YOKOYAMA, Keigo, Tokyo 146-8501 (JP); SATO, Sho, Tokyo 146-8501 (JP); TAMURA, Toshikazu, Tokyo 146-8501 (JP)
(74) Representative: TBK
(86) International application number: PCT/JP2010/054716
(87) International publication number: WO 2010/125871

(56) References cited:
- FR-A1- 2 851 359
- JP-A- 11 128 213
- JP-A- 2008 167 846
- US-A1- 2005 175 254
- US-A1- 2008 237 507
- US-B1- 6 343 112

## Description

### TECHNICAL FIELD

The present invention relates to an imaging apparatus and an imaging system, and a control method and a program for the same. More specifically, the present invention relates to a radiation imaging apparatus and a radiation imaging system, and a control method and a program for the same preferably used for still image radiographing such as radiography and moving image radiographing such as fluoroscopic imaging in medical diagnosis. Note that the term radiation according to the present invention should include not only α radiation, β radiation, and y radiation which are beams composed of particles (including photons) released by radioactive decay but also beams having the same or more energy such as X rays, particle radiation, and cosmic rays.

### BACKGROUND ART

As an imaging apparatus for use in medical image diagnosis and non-destructive inspection using X rays, recent years have seen the practical application of a radiation imaging apparatus using a flat panel detector (FPD) formed of semiconductor materials. For example, in medical image diagnosis, the radiation imaging apparatus has been used as a digital imaging apparatus for still image radiographing such as radiography and moving image radiographing such as fluoroscopic imaging.

With regard to such a radiation imaging apparatus, Patent Citation 1 investigates a radiation imaging apparatus capable of switching the FPD-read area (size of field of view) and the X-ray irradiation area. However, when switching is made so as to widen the irradiation area, the pixel sensitivity and the output under a dark state are different between the irradiated area and the non-irradiated area of the FPD. Consequently, a ghost (step of image) affected by the irradiation area occurs in the acquired image, which may lead to degradation of image quality.

To avoid such a ghost affected by the irradiation area, Patent Citation 2 investigates performing image processing for correction. Specifically, a ghost correction coefficient is calculated for each X-ray irradiation condition based on ghost data obtained by uniform irradiation. The ghost correction coefficient is used to acquire a required ghost correction coefficient corresponding to the X-ray irradiation condition and the elapsed time from the start of the X-ray irradiation when data about the target portion of inspection object, which is the irradiation area, is collected. Then, correction image data is generated by correcting the data about the target portion of inspection object by the required ghost correction coefficient.
Patent Citation 1: Japanese Patent Application Laid-Open No. H11-128213
Patent Citation 2: Japanese Patent Application Laid-Open No. 2008-167846

### DISCLOSURE OF THE INVENTION

### Technical Problem

However, the correction technique disclosed in Patent Citation 2 uses image processing for correction, and thus involves complicated parameter management and correction processing, thereby complicating the entire apparatus. In addition, data acquisition for correction is required in advance, leading to complicated operation. Moreover, in order to obtain stable image quality, strict management needs to be in place for the data collection method, leading to difficult management. Further, the correction technique does not reduce the after-image quantity itself which causes the above ghost and is contained in an image signal obtained from the FPD, and thus it is difficult to obtain an optimal effect under various circumstances.

### Solution of Problem

The present inventors have made zealous studies so as to provide an imaging apparatus and a system which can reduce a step of image which may occur in an obtained image and is affected by an irradiation area and can prevent remarkable degradation of image quality without performing complicated image processing. As a result of zealous studies, the inventors have envisioned the embodiments as described below.

*The object is solved by what is defined in the appended independent claims. Advantageous modifications thereof are set forth in the appended dependent claims.*

### Advantageous Effects of Invention

The present invention can reduce a ghost (step of image) which may occur in an obtained image and is affected by an irradiation area and can prevent remarkable degradation of image quality by drive operation of an FPD without performing complicated image processing.

Further features of the present invention will become apparent from the following description of exemplary embodiments with reference to the attached drawings.

### BRIEF DESCRIPTION OF THE DRAWINGS

FIG. 1 is a schematic block diagram of an imaging system including an imaging apparatus according to a first embodiment of the present invention.
FIG. 2 is a schematic equivalent circuit diagram of the imaging apparatus according to the first embodiment of the present invention.
FIG. 3 is a flowchart illustrating an operation of the imaging apparatus and the imaging system according to the present invention.
FIGS. 4A, 4B, 4C, and 4D each are a timing chart describing an operation of the imaging apparatus and the imaging system according to the present invention.
FIGS. 5A, 5B, and 5C are schematic views illustrating a configuration of a controlling computer of the present invention and a characteristic chart of integral dose versus dark output for describing the concept and advantages of the present invention.
FIGS. 6A and 6B each are a schematic equivalent circuit diagram of the imaging apparatus according to a second embodiment of the present invention.
FIGS. 7A, 7B, 7C, and 7D each are a timing chart describing an operation of the imaging apparatus and the imaging system according to the second embodiment of the present invention.
FIGS. 8A, 8B, and 8C each are a timing chart describing an operation according to the second embodiment of the present invention.

### BEST MODES FOR CARRYING OUT THE INVENTION

Hereinafter, embodiments to which the present invention can be preferably applied will be described in detail by referring to the accompanying drawings.

### (First Embodiment)

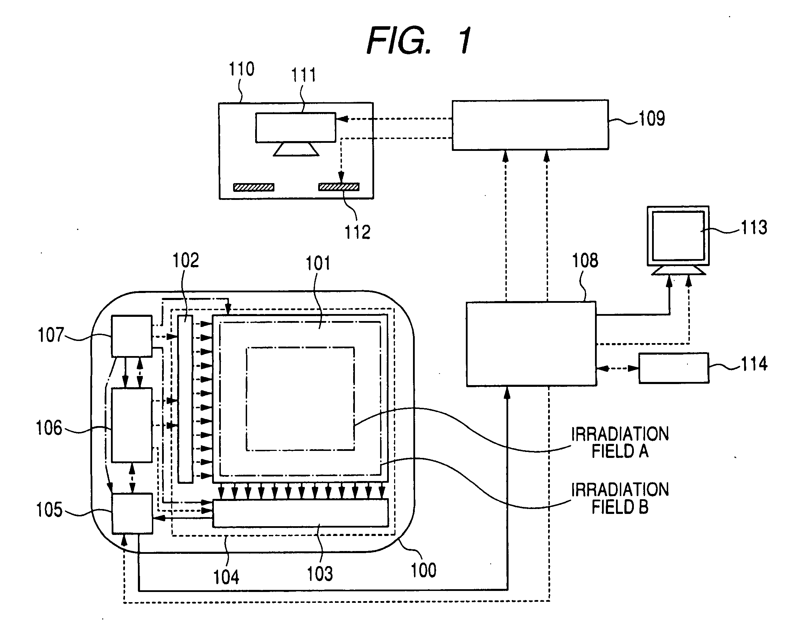

The radiation imaging system of the present embodiment illustrated in FIG. 1 includes an imaging apparatus 100, a controlling computer 108, a radiation control apparatus 109, a radiation generating apparatus 110, a display apparatus 113, and a control console 114. The imaging apparatus 100 has an FPD (flat panel detector) 104 which includes a detector unit 101 having a plurality of pixels each converting radiation or light to electrical signal; a driving circuit 102 driving the detector unit 101; and a read out circuit 103 outputting an electrical signal from the driven detector unit 101 as image data. The imaging apparatus 100 further includes a signal processing unit 105 which processes and outputs image data from the FPD 104; a controller unit 106 which controls an operation of the FPD 104 by supplying a control signal to each component; and a power source unit 107 which supplies bias to each component. The signal processing unit 105 which receives a control signal from the later described controlling computer 108 and provides the control signal to the controller unit 106. The power source unit 107 includes a power supply circuit such as a regulator which receives voltage from an external power source and a built-in battery (not illustrated) and supplies voltage required for the detector unit 101, the driving circuit 102, and the read out circuit 103.

The controlling computer 108 performs synchronization between the radiation generating apparatus 110 and the imaging apparatus 100; transmission of a control signal for determining a state of the imaging apparatus 100; and image processing for correction, storage, and display on image data from the imaging apparatus 100. In addition, the controlling computer 108 transmits, to the radiation control apparatus 109, a control signal determining a radiation irradiation condition based on information from the control console 114.

In response to a control signal from the controlling computer 108, the radiation control apparatus 109 controls an operation of a radiation source 111 emitting radiation therefrom and an operation of an irradiation field aperture mechanism 112, which are included in the radiation generating apparatus 110. The irradiation field aperture mechanism 112 has a function capable of changing a predetermined irradiation field which is an area for irradiating the detector unit 101 of the FPD 104 with radiation or light according to radiation. According to the present embodiment, the irradiation field aperture mechanism 112 has a function capable of switching between an irradiation field A and an irradiation field B. The irradiation field A corresponding to the first irradiation field of the present invention is irradiated with radiation corresponding to part of pixels contained in a plurality of pixels, assuming that, for example, the plurality of pixels is a total of pixels of about 2800 rows x about 2800 columns and the part of pixels is about 1000 rows x about 1000 columns. The irradiation field B corresponding to the second irradiation field of the present invention is irradiated with radiation corresponding to all the pixels wider than the irradiation field A. The control console 114 inputs examinee information and imaging conditions as parameters for various control of the controlling computer 108 and transmits the parameters to the controlling computer 108. The display apparatus 113 displays image data undergoing image processing by the controlling computer 108.

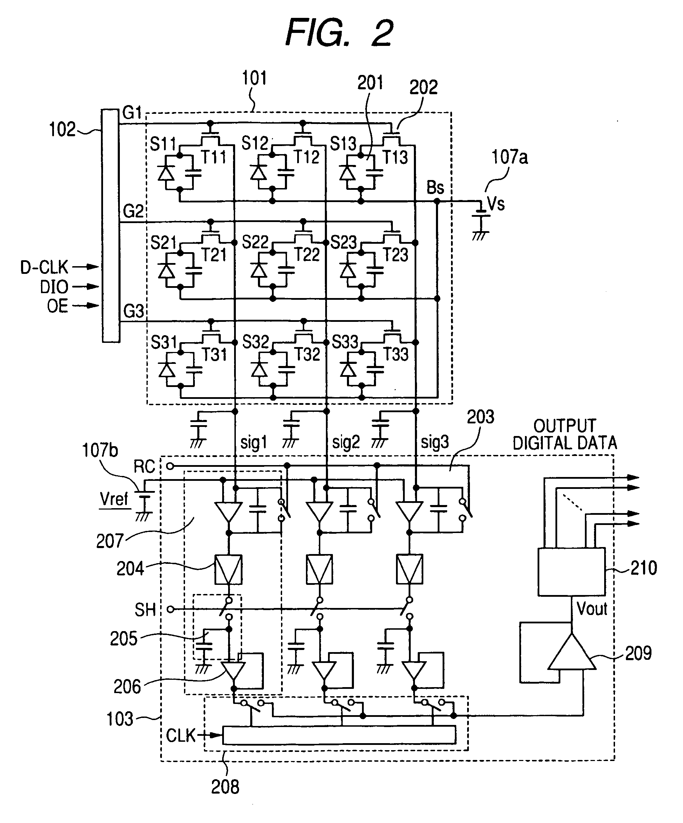

Next, by referring to FIG. 2, the imaging apparatus according to the first embodiment of the present invention will be described. Note that the same reference numerals or characters are assigned to the same components as those in FIG. 1, and the description thereof is omitted. For ease of description, FIG. 2 illustrates the imaging apparatus including the FPD having pixels of 3 rows × 3 columns. Note that in fact, an actual imaging apparatus has more pixels. For example, a 17-inch imaging apparatus has pixels of about 2800 rows × about 2800 columns.

The detector unit 101 has a plurality of pixels arranged in a matrix form. Each pixel has a conversion element 201 converting radiation or light to electric charge; and a switching element 202 outputting an electrical signal according to the electric charge. According to the present embodiment, as a photoelectric conversion element converting light emitted to a conversion element to electric charge, a PIN photodiode mainly composed of amorphous silicon arranged on an insulating substrate such as a glass substrate is used. As the conversion element, an indirect conversion element having a wavelength converter provided on a radiation incident side of the above described photoelectric conversion element, which converts radiation into light on a wavelength band sensible by the photoelectric conversion element and a direct conversion element which directly converts radiation into an electric charge are preferably used. As the switching element 202, a transistor having a control terminal and two main terminals are preferably used. According to the present embodiment, a thin-film transistor (TFT) is used. One electrode of the conversion element 201 is electrically connected to one of the two main terminals of the switching element 202, and the other electrode is electrically connected to a bias power source 107a via a common bias line Bs. The control terminals of a plurality of switching elements in rows such as T11 to T13 are electrically connected commonly to a drive line G1 in the first row, and a drive signal for controlling a conducting state of the switching elements is provided from the driving circuit 102 in units of rows via the drive line. The other main terminal of the control terminals of a plurality of switching elements in columns such as T11 to T31 are electrically connected to a signal line Sig 1 in the first column and outputs an electrical signal according to the electric charge of the conversion element to the read out circuit 103 via the signal line while the switching element is in a conducting state. The plurality of signal lines Sig 1 to Sig 3 provided in columns transmit electrical signals outputted from a plurality of pixels to the read out circuit 103 in parallel.

The read out circuit 103 includes an amplifier circuit 207 amplifying an electrical signal outputted in parallel from the detector unit 101, and the amplifier circuit 207 is provided for each signal line. Each amplifier circuit 207 includes an integration amplifier 203 amplifying an outputted electrical signal; a variable amplifier 204 amplifying the electrical signal from the integration amplifier 203; a sample hold circuit 205 sampling and holding the amplified electrical signal; and a buffer amplifier 206. The integration amplifier 203 includes an arithmetic amplifier amplifying and outputting the read electrical signal; an integration capacitor; and a reset switch. The integration amplifier 203 can change the amplification ratio by changing the integration capacitor value. The outputted electrical signal is inputted to an inverting input terminal of the arithmetic amplifier. A reference voltage Vref is inputted to a non-inverting input terminal from the reference power source 107b, and the amplified electrical signal is outputted from the output terminal. The integration capacitor is located between the inverting input terminal and the output terminal of the arithmetic amplifier. The sample hold circuit 205 is provided for each amplifier circuit and includes a sampling switch and a sampling capacitor. The read out circuit 103 includes a multiplexer 208 which sequentially outputs the electrical signal read in parallel from each amplifier circuit 207 and outputs a serial image signal; and a buffer amplifier 209 which performs impedance conversion on the image signal to be outputted. An image signal Vout, which is an analog electrical signal outputted from the buffer amplifier 209, is converted to digital image data by the A/D converter 210 and is outputted to the signal processing unit 105. The image data processed by the signal processing unit 105 illustrated in FIG. 1 is outputted to the controlling computer 108.

In response to the control signals (D-CLK, OE, and DIO) inputted from the controller unit 106 illustrated in FIG. 1, the driving circuit 102 outputs, to each drive line, the drive signal having a conducting voltage Vcom for placing the switching element in a conducting state and a non-conducting voltage Vss for placing the switching element to a non-conducting state. Thereby, the driving circuit 102 controls the conducting state and the non-conducting state of the switching element and drives the detector unit 101.

The power source unit 107 illustrated in FIG. 1 includes the bias power source 107a and the reference power source 107b of the amplifier circuit illustrated in FIG. 2. The bias power source 107a supplies a bias voltage Vs commonly to the other electrode of each conversion element via the bias line Bs. The bias voltage Vs corresponds to the first voltage of the present invention. The reference power source 107b supplies a reference voltage Vref to a non-inverting input terminal of each arithmetic amplifier.

The controller unit 106 illustrated in FIG. 1 receives control signals from the controlling computer 108 and other external devices via the signal processing unit 105 and controls the operation of the FPD 104 by giving various control signals to the driving circuit 102, the power source unit 107, and the read out circuit 103. The controller unit 106 controls the operation of the driving circuit 102 by providing the driving circuit 102 with the control signal D-CLK, the control signal OE, and the control signal DIO. Here, the control signal D-CLK is a shift clock for a shift register used as the driving circuit; the control signal DIO is a pulse transferred by the shift register; and the control signal OE is a control signal for controlling an output end of the shift register. The controller unit 106 controls the operation of various components of the read out circuit 103 by providing the read out circuit 103 with a control signal RC, a control signal SH, and a control signal CLK. Here, the control signal RC is a control signal for controlling the operation of the reset switch of the integration amplifier; the control signal SH is a control signal for controlling the operation of the sample hold circuit 205; and the control signal CLK is a control signal for controlling the operation of the multiplexer 208.

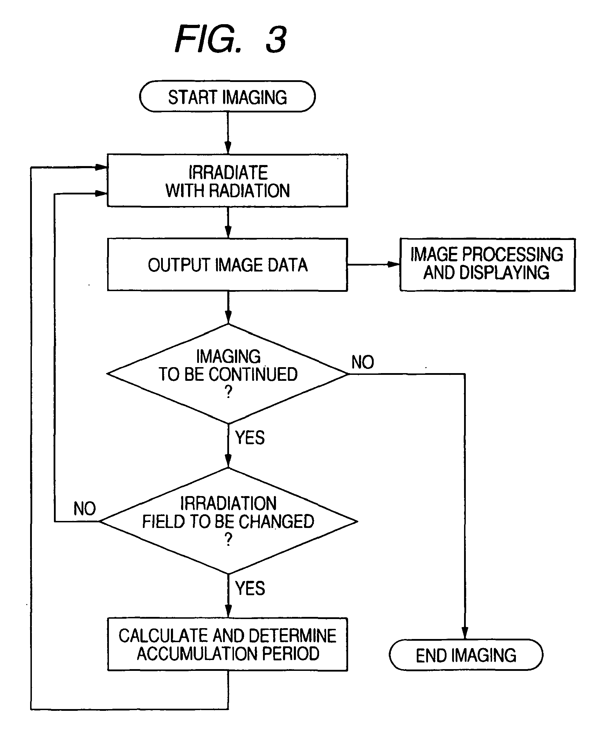

Next, by referring to FIGS. 1 to 3, particularly FIG. 3, the entire operation of the imaging apparatus and the imaging system of the present invention will be described. The operator operates the control console 114; the controlling computer 108 determines the irradiation conditions; and imaging starts. Then, a desired dose of radiation is delivered to an examinee from the radiation generating apparatus 110 controlled by the radiation control apparatus 109 under the irradiation conditions. The imaging apparatus 100 outputs image data according to the radiation transmitted through the examinee. The outputted image data undergoes image processing by the controlling computer 108 and is displayed on the display apparatus 113.

The controlling computer 108 prompts the operator to confirm whether or not to continue imaging. If an instruction of no imaging continuation (NO) is received from the operator, the controlling computer 108 terminates imaging; and if an instruction of imaging continuation (YES) is received from the operator, the controlling computer 108 prompts the operator to confirm whether or not to change the irradiation field. If an instruction of no irradiation field change (NO) is received from the operator, the controlling computer 108 controls the radiation control apparatus 109 and the radiation generating apparatus 110 under the previously determined imaging conditions so that radiation is delivered again under the same conditions. Conversely, if an instruction of irradiation field change (YES) is received from the operator, the controlling computer 108 determines the irradiation conditions where the irradiation field is changed. Based on the determined irradiation conditions, the radiation control apparatus 109 controls the irradiation field aperture mechanism 112 of the radiation generating apparatus 110 so as to determine the changed irradiation field. In addition, the controlling computer 108 performs an arithmetic processing for determining an accumulation period described in detail later. Subsequently, the controlling computer 108 controls the radiation control apparatus 109 and the radiation generating apparatus 110 under the irradiation conditions including the irradiation field determined by the controlling computer 108 and the irradiation time according to the accumulation period so as to deliver radiation under the changed irradiation conditions. In addition, the controlling computer 108 provides the imaging apparatus 100 with the control signal based on the determined accumulation period and the next imaging is performed under the determined accumulation period.

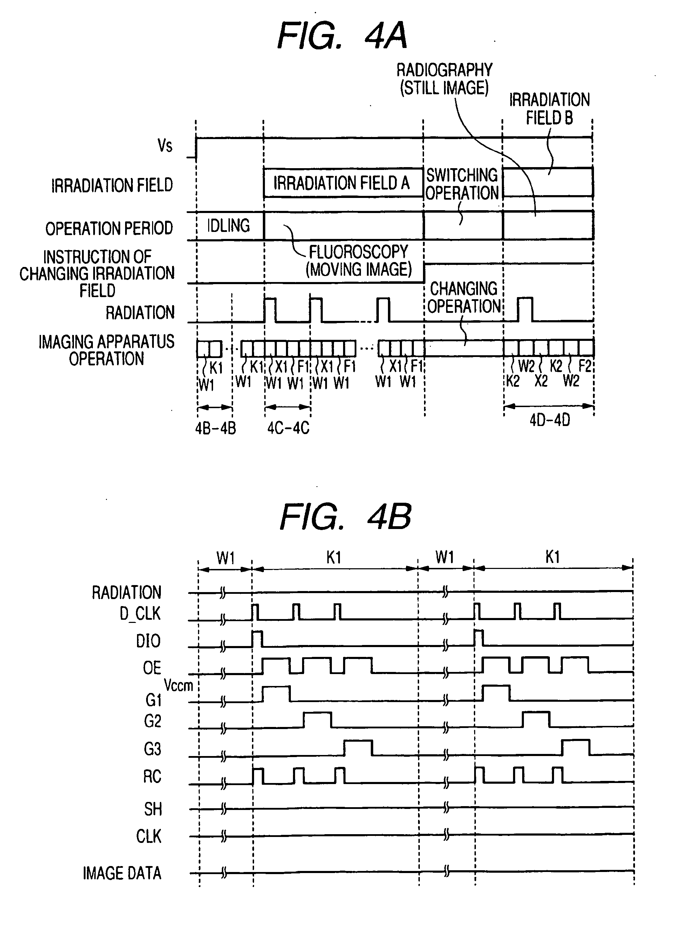

Next, by referring to FIGS. 4A to 4D, the operation of the imaging system of the present invention will be described. In FIG. 4A, when the bias voltage Vs is supplied to the conversion element 201, the imaging apparatus 100 performs an idling operation in the idling period. Here, the idling operation means an operation for repeatedly performing at least an initialization operation K1 a plurality of times in order to stabilize the characteristic variation of the detector 104 caused by the start of applying the bias voltage Vs. The initialization operation means an operation for initializing a conversion element by providing the conversion element with an initial bias before the accumulation operation. Note that in FIG. 4A, as the idling operation, a pair of the accumulation operation W1 and the initialization operation K1 is repeatedly performed a plurality of times.

FIG. 4B is a timing chart describing the operation of the imaging apparatus in the period 4B-4B illustrated in FIG. 4A. As illustrated in FIG. 4B, in the accumulation operation W1, the bias voltage Vs is supplied to the conversion element 201; in that state, the non-conducting voltage Vss is supplied to the switching element 202; and thus the switching elements of all the pixels are placed in the non-conducting state. In the initialization operation K1, first, the reset switch causes the integration capacitor of the integration amplifier and the signal line to be reset; then, the conducting voltage Vcom is supplied to the drive line G1 from the driving circuit 102; and thus the switching elements T11 to T13 of the pixels in the first row are placed in the conducting state. When the switching elements are placed in the conducting state, the conversion elements are initialized. At this time, the electric charge of the conversion element is outputted as an electrical signal by the switching element. However, according to the present embodiment, the circuits following the sample hold circuit are not operated, and thus data according to the electrical signal is not outputted from the read out circuit 103. Subsequently, when the integration capacitor and the signal line are reset again, the outputted electrical signal is processed. Note that in order to use the data for correction and other purpose, the circuits following the sample hold circuit may be operated in the same manner as an image output operation and a dark image output operation described later. In this manner, the initialization operation of the detector 101 is performed by repeating the control and reset of the conducting state of the switching element in the second row, in the third row, and so on. Here, in the initialization operation, during the period while at least the switching element is in the conducting state, the reset switch may be kept in the conducting state so as to continue reset. In addition, the conducting time of the switching element in the initialization operation may be shorter than the conducting time of the switching element in the image output operation described later. Moreover, in the initialization operation, a plurality of rows of switching elements may be conducted at the same time. By doing so, it is possible to shorten the time required for the entire initialization operation and to hasten stabilization of the characteristic variation of the detector. Note that the initialization operation K1 of the present embodiment is performed in the same period as that of the image output operation included in the fluoroscopic imaging operation performed after the idling operation.

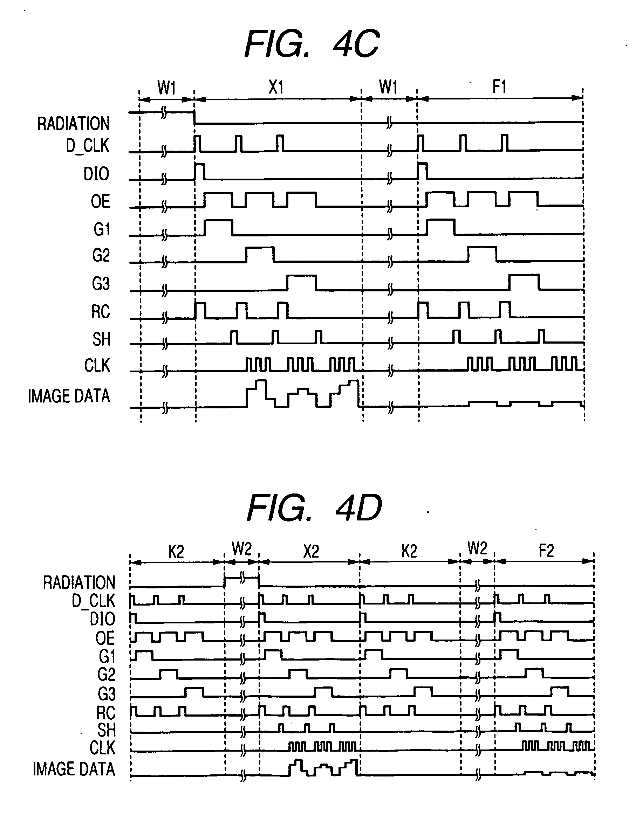

FIG. 4C is a timing chart describing the operation of the imaging apparatus in the period 4C-4C illustrated in FIG. 4A. After the idling operation is performed, the detector 101 enters a state capable of imaging. Then, in response to a control signal from the controlling computer 108, the imaging apparatus 100 performs a fluoroscopic imaging operation of emitting radiation to the FPD 104 in an area of the irradiation field A. The fluoroscopic imaging operation corresponds to the first imaging operation of the present invention. The period while the imaging apparatus 100 is performing the fluoroscopic imaging operation is referred to as the fluoroscopic imaging period. During the fluoroscopic imaging period, so that the conversion element 201 generates an electric charge according to the emitted radiation, the imaging apparatus 100 performs an accumulation operation W1 performed in a period according to the time to emit radiation; and an image output operation X1 of outputting image data based on the electric charge generated by the accumulation operation W1. As illustrated in FIG. 4C, in the image output operation, first, the integration capacitor and the signal line are reset; then, the conducting voltage Vcom is supplied to the drive line G1 from the driving circuit 102; and thus the switching elements T11 to T13 in the first row are placed in the conducting state. Thereby, an electrical signal based on the electric charge generated by the switching elements S11 to S13 in the first row is outputted to each signal line. Each of the electrical signals outputted in parallel via each signal line is amplified by the arithmetic amplifier 203 and the variable amplifier 204 of each amplifier circuit 206. Then, the control signal SH causes the sample hold circuit to be operated and each of the amplified electrical signals is held in parallel in the sample hold circuit 205 inside each amplifier circuit. After being held, the integration capacitor and the signal line are reset. After being reset, in the same manner as in the first row, the conducting voltage Vcom is supplied to the drive line G2 in the second row, and thus the switching elements T21 to T23 in the second row are placed in the conducting state. Within the period while the switching elements T21 to T23 in the second row are placed in the conducting state, the multiplexer 208 sequentially outputs the electrical signal held in the sample hold circuit 205. Thereby, the electrical signals from the pixels in the first row read in parallel are converted to a serial image signal to be outputted. Then, the A/D converter 210 converts the serial image signal to a row of image data to be outputted. The above operation is performed in units of rows from the first row to the third row, and a frame of image data is outputted from the imaging apparatus. Further, according to the present embodiment, so that the conversion element 201 generates an electric charge in a dark state where no radiation is emitted, the imaging apparatus 100 performs the accumulation operation W1 performed in the same period as that of the accumulation operation W1 and a dark image output operation F1 of outputting dark image data based on the electric charge generated by the accumulation operation W1. In the dark image output operation F1; the same operation as the image output operation X1 is performed by the imaging apparatus 100.

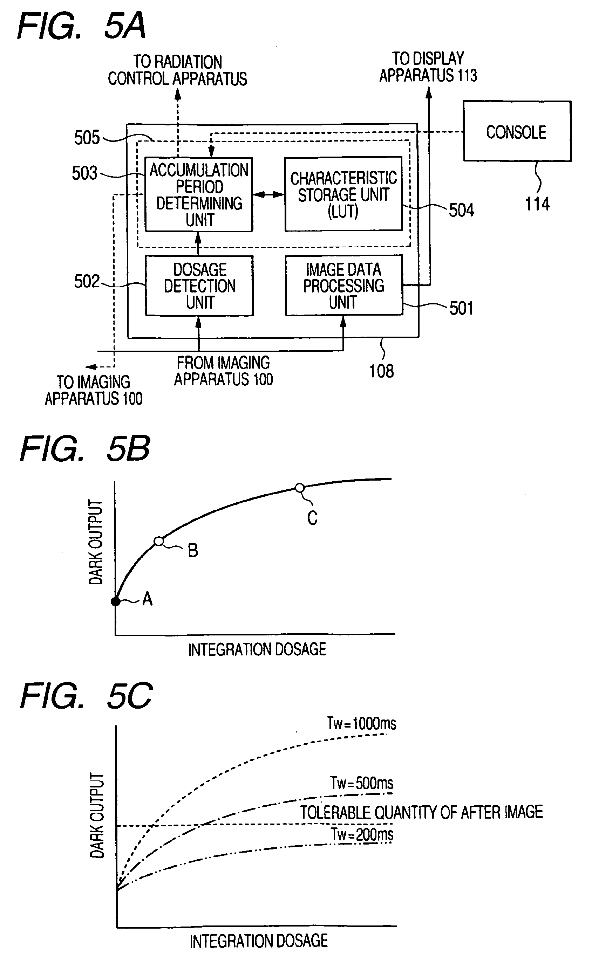

Next, when an irradiation field change instruction is transmitted from the control console 114 to the controlling computer 108, the controlling computer 108 accordingly performs an arithmetic processing to determine the accumulation period. The period while the arithmetic processing is being performed is referred to as the arithmetic period. The arithmetic processing will be described in detail later by referring to FIGS. 5A to 5C.

FIG. 4D is a timing chart describing the operation of the imaging apparatus in the period 4D-4D illustrated in FIG. 4A. After the arithmetic processing, the controlling computer 108 provides the imaging apparatus 100 with a control signal according to the accumulation period determined by the arithmetic processing. Based on the control signal from the controlling computer, the imaging apparatus 100 performs radiography (still image) operation in which radiation is emitted to the FPD 104 in the irradiation field B whose area is wider than the area of the irradiation field A. The radiography operation corresponds to the second imaging operation of the present invention. The period while the imaging apparatus 100 is performing the radiography operation is referred to as the radiography period. In the radiography period, so that the conversion element generates an electric charge according to the emitted radiation, the imaging apparatus 100 performs an accumulation operation W2 performed in the accumulation period Tw determined by the arithmetic processing and an image output operation X2 of outputting image data based on the electric charge generated by the accumulation operation W2. As illustrated in FIG. 4D, here, according to the present embodiment, the accumulation operation W2 and the image output operation W2 are the same as the accumulation operation W1 and the image output operation W1 respectively, but according to the present embodiment, each operation is different in period, and thus different reference characters are assigned respectively. Note that each operation may be performed in the same period depending on the results of the arithmetic processing. Moreover, according to the present embodiment, so that the conversion element generates an electric charge in a dark state where no radiation is emitted, the imaging apparatus 100 performs the accumulation operation W2 performed in the same period as that of the accumulation operation W2 and a dark image output operation F2 of outputting dark image data based on the electric charge generated by the accumulation operation W2. In the dark image output operation F2, the same operation as the image output operation X2 is performed by the imaging apparatus 100. Further, according to the present embodiment, the imaging apparatus 100 performs the initialization operation K2 before each accumulation operation W2. Here, the initialization operation K2 is the same as the previously described initialization operation K1, but according to the present embodiment, each operation is different in period, and thus different reference characters are assigned. Note that like the above described accumulation period W2, each operation may be performed in the same period depending on the results of the arithmetic processing.

Next, by referring to FIGS. 5A to 5C, the arithmetic processing by the controlling computer which is a control means of the present embodiment will be described. Note that in FIGS. 5B and 5C, the horizontal axis indicates an integral dose of radiation emitted to the FPD 104. The vertical axis indicates output data of a pixel obtained in a dark state as a dark output. Note that according to the present embodiment, an area of the detector to which radiation is emitted in the irradiation field A is referred to as a first area; and an area of the detector to which radiation is emitted in the irradiation field B and which excludes the first area is referred to as a second area.

First, by referring to FIGS. 5B and 5C, a mechanism of generating a step of image, which serves as a base of the arithmetic processing of the present invention, will be described. As illustrated in FIG. 5B, the present inventors have found that the dark output of the flat panel detector depends on a radiation irradiation history, and more specifically, depends on an integral dose of radiation after a bias voltage is applied to a conversion element of the flat panel detector. According to the present embodiment, the imaging operation is performed in the irradiation field A. Thus, the dark output of a pixel contained in the second area is indicated by A in FIG. 5B; and the dark output of a pixel contained in the first area is indicated by B or C. The dark output of a pixel contained in the first area depends on the integral dose which depends on the length of a fluoroscopic imaging operation period, and thus the dark output thereof is as indicated by B or C in FIG. 5B. Therefore, for example, a difference occurs between the dark output A in the second area and the dark output C in the first area, and the difference in dark output causes a step of image. In particular, the longer the fluoroscopic imaging operation period, the larger the difference in dark output between the first area and the second area, and thus the more apparent the step of image. Thus, the present inventors have found that the dark output of the flat panel detector depends on the radiation irradiation history; accordingly, a difference in dark output occurs between an irradiated area of radiation and a non-irradiated area in the flat panel detector; and the difference causes a step of image to occur.

As illustrated in FIG. 5C, the dark output of the flat panel detector depends on the accumulation period Tw of the conversion element. Thus, the present inventors have found that the step of image depends on the integral dose of radiation before the irradiation field is changed and the accumulation period Tw of the conversion element in the imaging operation after the irradiation field is changed. In addition, the present inventors have found that if the step of image is a predetermined tolerable quantity or less, no step of image is recognized and thus the image obtained from the imaging apparatus can be used. Note that the predetermined tolerable quantity is a value specific to the detector, and thus can be preliminarily acquired and set before inspection at shipping. In particular, if the step of image is equal to or less than the random noise of the flat panel detector, the step of image generally is lost in the random noise and cannot be recognized as the step of image; and thus it is desirable that the tolerable quantity is equal to or less than the random noise output.

As described above, based on the information about the integral dose of radiation in the imaging operation before the irradiation field is changed, the controlling computer 108 performs an arithmetic processing to determine the accumulation period in the imaging operation after the irradiation field is changed so that the step of image is equal to or less than a preliminarily set tolerable quantity. Thereby, the upper limit of the accumulation period is the same time as the preliminarily set tolerable quantity of the step of image. Note that the radiation generating apparatus 112 must emit a dose of radiation required for imaging in the time width within the accumulation period. If the accumulation period is made too short, in some cases, the dose of radiation required for imaging cannot be secured unless the imaging is performed in an extremely short time or with an extremely strong intensity exceeding the limit of the radiation generating apparatus. In other words, the time period when the radiation generating apparatus 112 can emit a dose of radiation required for imaging is the lower limit of the accumulation period. For this reason, the controlling computer 108 determines the accumulation period so that the step of image is equal to or less than the preliminarily set tolerable quantity within a range in which the radiation generating apparatus can emit a dose of radiation required for imaging operation after the irradiation field is changed. Note that if the result obtained by the arithmetic processing is a significantly shorter time than the limit of the radiation generating apparatus, the lower limit of the accumulation period is the shortest irradiation time which is the time limit allowing the radiation generating apparatus to generate radiation. In that case, in order to secure a dose of radiation required for imaging, the controlling computer 108 controls the radiation generating apparatus so as to increase the radiation to be emitted. Specifically, the controlling computer 108 controls the radiation intensity by controlling a tube current of the radiation source of the radiation generating apparatus.

Then, the controlling computer 108 provides the controller unit of the imaging apparatus with a control signal based on the determined accumulation period, and controls the driving circuit so that the controller unit performs an accumulation operation of the detector in the determined accumulation period. In addition, the controlling computer 108 provides the radiation control apparatus with a control signal based on the determined accumulation period, and controls the radiation generating apparatus so that the radiation generating apparatus emits a dose of radiation required for imaging operation after the irradiation field is changed according to the determined accumulation period.

Next, by referring to FIG. 5A, a configuration for performing the arithmetic processing of the present invention and a specific arithmetic processing will be described. The controlling computer 108 includes an image data processing unit 501, a dose detection unit 502, an accumulation period determining unit 503, and a characteristic storage unit 504. Here, the characteristic storage unit 504 stores data about the integral dose, the accumulation period, and the dark output indicating characteristics of the detector, and a lookup table containing these kinds of data is preferably used. In addition, information about the shortest irradiation time and the maximum output intensity of the radiation generating apparatus is stored in the characteristic storage unit 504. According to the present invention, the arithmetic processing unit 505 includes the accumulation period determining unit 503 and the characteristic storage unit 504.

The image data outputted from the imaging apparatus 100 undergoes image processing by the image data processing unit 501, and is transmitted to the display apparatus 113. Of the image data, the image data corresponding to a pixel contained in the first area is transmitted to the dose detection unit 502 as dose detection data. The dose detection unit 502 calculates the radiation dose for each frame based on the dose detection data to be accumulated. Here, as the dose detection data, image data corresponding to a specific pixel contained in the first area may be used or an average value of the image data outputted from a plurality of pixels contained in the first area may be used. Instead of image data, data from a photo timer (not illustrated) provided in the imaging apparatus separately from the detector unit may be used. The dose detection unit 502 obtains information about the integral dose in the imaging operation by adding the accumulated radiation dose for each frame and outputs the information to the accumulation period determining unit 503.

When the operator inputs an instruction to change the irradiation field to the control console 114, the control console 114 outputs, to the accumulation period determining unit 503, a control signal instructing the irradiation field to be changed and information about the radiation dose required for imaging after the irradiation field is changed. The accumulation period determining unit 503 which receives the control signal from the control console 114 determines the accumulation period Tw based on the information about the inputted integral dose, information about the required radiation dose, and data stored in the characteristic storage unit 504.

The determined accumulation period Tw is outputted from the accumulation period determining unit 503 to the controller unit 106 of the imaging apparatus 100. The controller unit 106 controls the driving circuit so as to perform an accumulation operation of the detector in the inputted accumulation period Tw. In addition, the accumulation period Tw and the information about the required radiation dose are transmitted from the accumulation period determining unit 503 to the radiation control apparatus 109. The radiation control apparatus 109 controls the radiation generating apparatus 112 so that the radiation generating apparatus 112 emits a dose of radiation required for imaging according to the accumulation period Tw.

As described above, the imaging operation after the irradiation field is changed is performed in the accumulation period determined based on the integral dose of radiation in the imaging operation before the irradiation field is changed, which can reduce a step of image affected by the irradiation area and can prevent remarkable degradation of image quality without performing complicated image processing. Note that according to the present embodiment, the accumulation period Tw is determined, but the present invention is not limited to this. For example, a control may be made in such a manner that the accumulation period Tw and the period of the initialization operation K2 performed immediately before the accumulation period Tw are combined; both the accumulation period Tw and the period of the initialization operation K2 are calculated and determined; and the output operation X2 and the like may be performed according to the initialization operation K2. Note also that according to the present embodiment, the controlling computer 108 performs the arithmetic processing, but the present invention is not limited to this. For example, in response to a control signal from the controlling computer, the controller unit 106 of the imaging apparatus 100 may perform the arithmetic processing.

### (Second Embodiment)

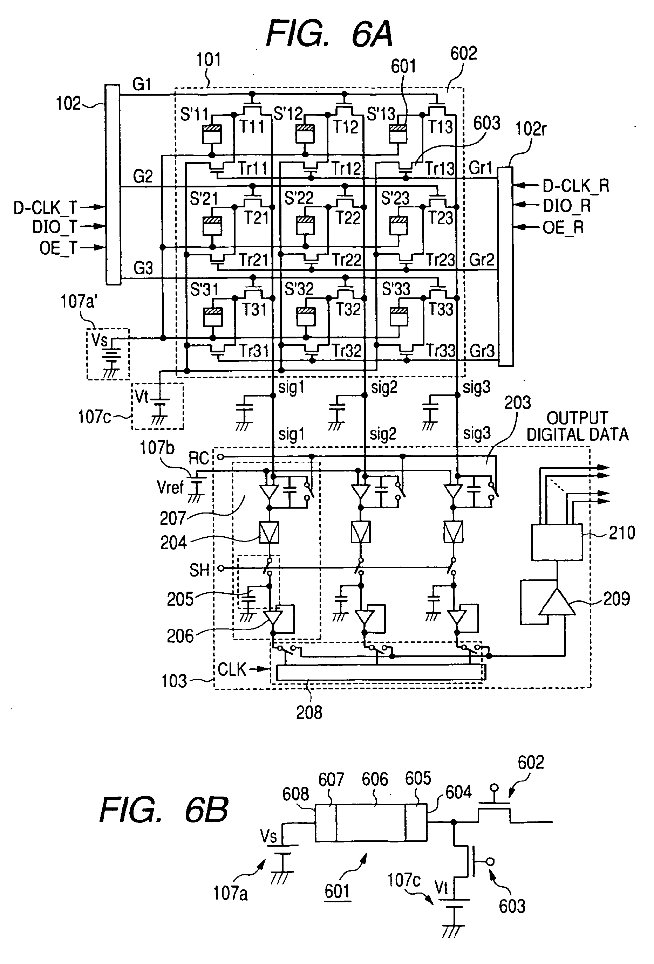

Next, by referring to FIGS. 6A and 6B, the imaging apparatus according to the second embodiment of the present invention will be described. Note that the same reference numerals or characters are assigned to the same components as those in the first embodiment, and the detailed description thereof is omitted. For ease of description, FIG. 6A illustrates the imaging apparatus including the FPD having pixels of 3 rows × 3 columns in the same configuration as in FIG. 2, but in fact, an actual imaging apparatus has more pixels.

According to the detector unit 101 of the first embodiment, a PIN photodiode is used in the conversion element 201, while according to the detector unit 101' of the present embodiment, an MIS photoelectric conversion element is used in the conversion element 601 as the MIS conversion element. According to the first embodiment, a switching element for outputting is provided for each pixel; while according to the present embodiment, in addition to the switching element 602 for outputting, a switching element 603 for refreshing is provided. One of the main terminals of the switching element 603 for refreshing is electrically connected to a first electrode 604 of the conversion element 601 and one of the two main terminals of the switching element 602. The other one of the main terminals of the switching element 603 is electrically connected to a refreshing power source 107c contained in the power source unit 107 via a common line. The control terminals of a plurality of switching elements 603 in rows are electrically connected commonly to a refreshing drive line Gr, and a drive signal is supplied from the refreshing driving circuit 102r in units of rows via the refreshing drive line Gr.

As illustrated in FIG. 6B, the conversion element 601 is configured such that a semiconductor layer 606 is provided between the first electrode 604 and a second electrode 608; an insulating layer 605 is provided between the first electrode 604 and the semiconductor layer 606; and an impurity semiconductor layer is provided between the semiconductor layer 606 and the second electrode 608. The second electrode 608 is electrically connected to a bias power source 107a' via the bias line Bs. In the same manner as the conversion element 201, the conversion element 601 is configured such that a bias voltage Vs is supplied to the second electrode 608 from the bias power source 107a'; a reference voltage Vref is supplied to the first electrode 604 via the switching element 602; and thus accumulation operation is performed. Here, in the fluoroscopic imaging operation and the radiography operation, a refreshing voltage Vt is supplied to the first electrode 604 via the switching element 603, and the conversion element 601 is refreshed by the bias |Vs-Vt|.

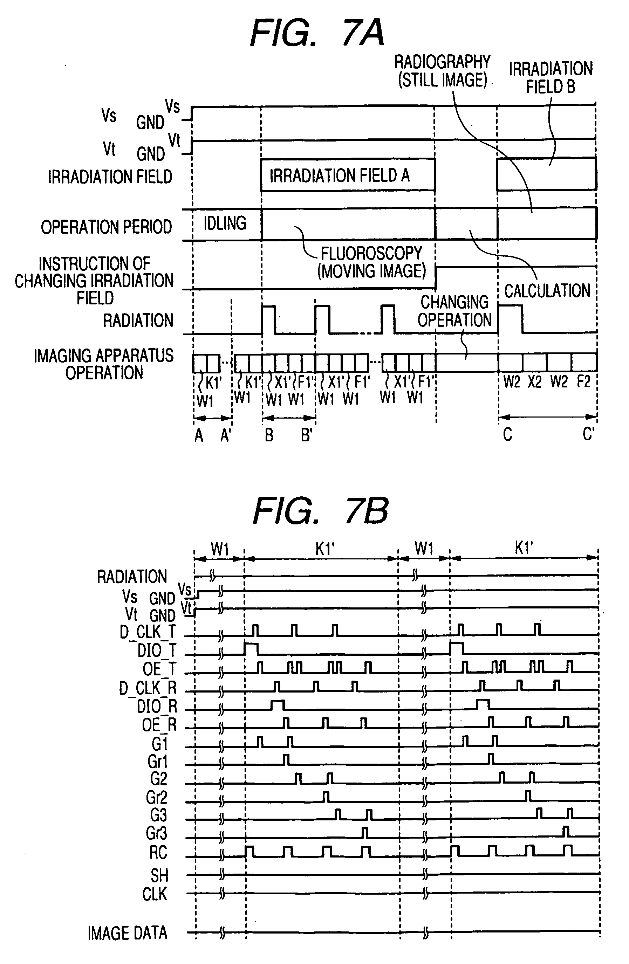

Next, by referring to FIGS. 7A to 7D, the operation of the imaging apparatus and the imaging system of the present embodiment will be described. According to the present embodiment illustrated in FIG. 7A, instead of the initialization operation K1, the image output operation X1, and the dark image output operation F1 of the first embodiment illustrated FIG. 4A, an initialization operation K1', an image output operation X1', and a dark image output operation F1' are performed respectively. In addition, instead of the image output operation X2 and the dark image output operation F2 of the first embodiment illustrated FIG. 4A, the image output operation X2' and the dark image output operation F2' are performed respectively. Further, according to the present embodiment, the imaging apparatus 100 performs a change operation described in detail later during the arithmetic period. The operation other than the above is the same as that of the first embodiment, and the detailed description thereof is omitted. Hereinafter, the different operations will be described by referring to FIGS. 7B to 7D.

According to the present embodiment, the configuration of a pixel includes not only the switching element 602 for outputting but also the switching element 603 for refreshing. For this reason, the initialization operation K1' in the idling operation of the present embodiment illustrated in FIG. 7B is different from the initialization operation K1 operated by one switching element 202 provided for one pixel. In the same manner as in the first embodiment, the initialization operation K1' is performed such that the conducting voltage Vcom is supplied to the drive line G from the driving circuit 102; then, the switching element 602 is placed in the conducting state; and the electric charge of the conversion element 601 is outputted as an electrical signal by the switching element 602. Subsequently, when the conducting voltage Vcom is supplied to the drive line Gr from the driving circuit 102r, the switching element 603 for refreshing is placed in the conducting state. At this time, a refreshing voltage Vt is supplied from the refreshing power source 107c. Thereby, the bias |Vs-Vt| is applied to the conversion element 601, and the residual charge in the conversion element is erased so as to refresh the conversion element. Then, the integration capacitor and the signal line are reset, and the switching element 602 is placed in the conducting state again. Then, an initial bias |Vs-Vref| is applied to the conversion element, and the conversion element is initialized. The initialization operation K1' is achieved by sequentially performing the above operation in units of rows. The operation other than the above is the same as that of the first embodiment, and the detailed description thereof is omitted.

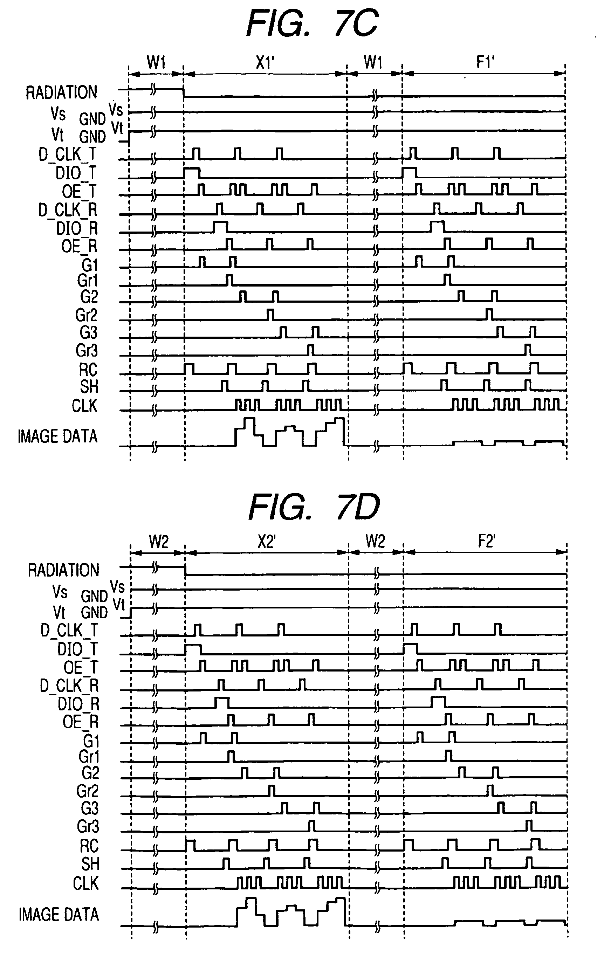

In addition, the difference between the image output operation X1' in the fluoroscopic imaging operation of the present embodiment illustrated in FIG. 7C and the image output operation X1, and the difference between the dark image output operation F1' and the dark image output operation F1 are the same as the above described difference between the initialization operation K1' and the initialization operation K1. The operation other than the above is the same as that of the first embodiment, and the detailed description thereof is omitted.

In the same manner as in the first embodiment, an image output operation X2' and a dark image output operation F2' in the radiography operation of the present embodiment illustrated in FIG. 7D are performed in such a manner that the conducting voltage Vcom is supplied to the drive line G from the driving circuit 102 and then the switching element 602 is placed in the conducting state. Thereby, the electric charge of the conversion element 601 is outputted in units of rows as an electrical signal by the switching element 602, and image data is outputted from the imaging apparatus via the read out circuit 103. Subsequently, when the conducting voltage Vcom is supplied to the drive line Gr from the driving circuit 102r, the switching element 603 for refreshing is placed in the conducting state. At this time, a refreshing voltage Vt is supplied from the refreshing power source 107c. Thereby, the bias |Vs-Vt| is applied to the conversion element 601, and the residual charge in the conversion element is erased so as to refresh the conversion element. Then, the integration capacitor and the signal line are reset, and the switching element 602 is placed in the conducting state again. Then, an initial bias |Vs-Vref| is applied to the conversion element, and the conversion element is initialized. The image output operation X2' and the dark image output operation F2' are achieved by sequentially performing the above operation in units of rows. Note that according to the present embodiment, the period of the image output operation X2' is longer and different than the period of the image output operation X1', and thus different reference characters are assigned to each operation, but the operation may be performed in the same period.

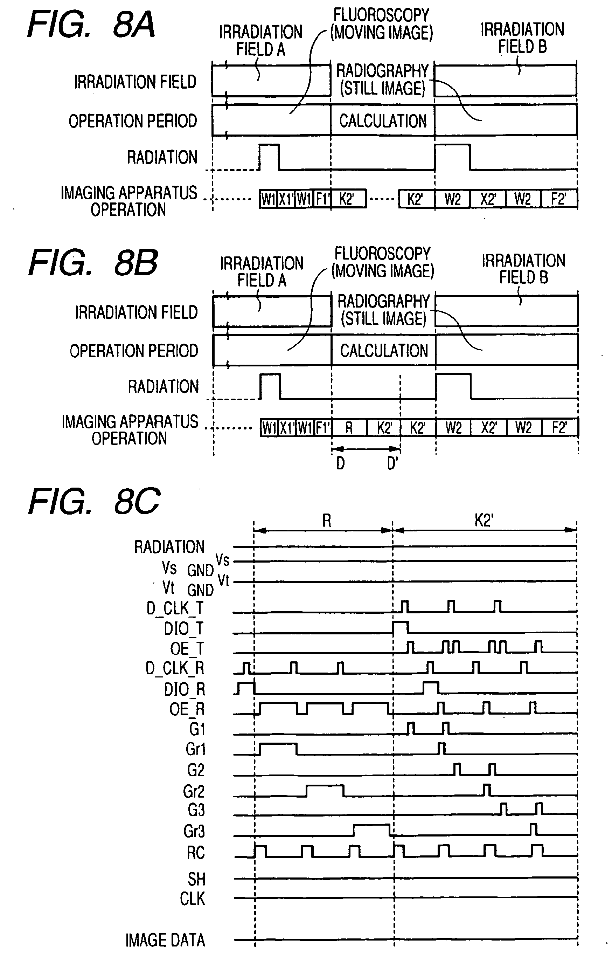

Next, by referring to FIGS. 8A to 8C, the change operation of the present embodiment will be described.

According to the change operation illustrated in FIG. 8A, the FPD 104 performs the initialization operation K2' once or a plurality of times in the same manner as in the initialization operation F1' in the same length of period as the output operations W2' and F2' of the radiography operation. In other words, the FPD 104 performs the initialization operation K2' once or a plurality of times corresponding to the output operations W2' and F2' of the radiography operation performed after the irradiation field is changed. According to the initialization operation K2', the change operation is performed by the initialization operation corresponding to an imaging operation performed after change, and thus excellent image data with few artifacts can be acquired. Since no accumulation operation is performed, the characteristics of the conversion element can be stabilized in a short time. In particular, as the change operation performed by a plurality of times of initialization operations, it is preferable that an initialization operation corresponding to an imaging operation performed after change is performed at least once immediately before the imaging operation performed after change.

According to the change operation illustrated in FIG. 8B, first, the FPD 104 performs a refresh operation R at least once as described later. Subsequently, the FPD 104 performs the initialization operation K2' once or a plurality of times corresponding to the output operations W2' and F2' of the radiography operation performed after the irradiation field is changed. In addition to the effect of the change operation illustrated in FIG. 8A, this change operation allows the refresh operation R to erase the residual charge in the conversion element, and thus can further reduce the step of image. Hereinafter, the refresh operation will be described by referring to FIG. 8C.

In the refresh operation illustrated in FIG. 8C, first, the driving circuit 102 does not supply the conducting voltage Vcom to the switching element 602, and thus the switching element 602 maintains the non-conducting state. In this state, the driving circuit 102r supplies the conducting voltage Vcom in units of rows to the switching element 603, and thus the switching element 603 is placed in the conducting state accordingly. Thereby, the bias |Vs-Vt| is applied to the conversion element 601, and the residual charge in the conversion element is erased so as to refresh the conversion element. The refresh operation R is achieved by sequentially performing the above operation in units of rows.

After the refresh operation R, the integration capacitor and the signal line are reset. Then, the conducting voltage Vcom is supplied to the drive line G from the driving circuit 102, and the switching element 602 is placed in the conducting state. Then, the electric charge of the conversion element 601 is outputted as an electrical signal by the switching element 602. Subsequently, when the conducting voltage Vcom is supplied to the drive line Gr from the driving circuit 102r, the switching element 603 for refreshing is placed in the conducting state. At this time, a refreshing voltage Vt is supplied from the refreshing power source 107c. Thereby, the bias |Vs-Vt| is applied to the conversion element 601, and the residual charge in the conversion element is erased so as to refresh the conversion element again. Then, the integration capacitor and the signal line are reset, and the switching element 602 is placed in the conducting state again. Then, an initial bias |Vs-Vref| is applied to the conversion element, and the conversion element is initialized. The initialization operation K2' is achieved by sequentially performing the above operation in units of rows.

Note that in the same manner as in the first embodiment, in the present embodiment, the second imaging operation may also include the initialization operation.

According to the present embodiment, in addition to performing the imaging operation after the irradiation field is changed in the accumulation period determined based on the integral dose of radiation in the imaging operation before the irradiation field is changed, the imaging apparatus 100 performs the change operation in the arithmetic period. Therefore, in addition to the effect of the first embodiment, the second embodiment can reduce the amount of step of image contained in the image data outputted from the imaging apparatus 100 and thus can further reduce the step of image.

It should be noted that each embodiment of the present invention can be implemented by programs to be executed by a computer such as the controller unit 106. Moreover, means for supplying the programs to the computer such as a computer-readable recording medium containing the programs such as a CD-ROM and a transmission medium for transmitting the programs such as the Internet can be applied as embodiments of the present invention. The above programs can also be applied as an embodiment of the present invention. The above programs, recording media, transmission media, and program products are included into the category of the present invention. In addition, any invention by a combination easily imaginable from the first or second embodiment is also included into the category of the present invention.

While the present invention has been described with reference to exemplary embodiments, it is to be understood that the invention is not limited to the disclosed exemplary embodiments. The scope of the following claims is to be accorded the broadest interpretation so as to encompass all such modifications and equivalent structures and functions.

## Claims

1. An imaging system comprising:
an imaging apparatus (100) comprising a detector (101) including a plurality of pixels arranged in a matrix, wherein each of the pixels includes a conversion element (201) for converting a radiation or a light into an electric charge, such that the detector (101) performs an imaging operation for outputting an image data based on an irradiation with the radiation or the light, and a controller unit (106) for controlling operations of the detector (101) including the imaging operation, wherein the imaging operation includes a first imaging operation for outputting an image data based on the irradiation with the light or the radiation on a first irradiation field (A) of the detector (101) corresponding to a part of the plurality of pixels, and a second imaging operation for outputting an image data based on the irradiation with the light or the radiation on a second irradiation field (B) of an area larger than that of the first irradiation field (A); and
a controlling computer (108) for controlling the imaging apparatus (100),
**characterized in that**
the controlling computer (108) supplies the controller unit (106) with a control signal based on an accumulation period determined based on an information relating to an integral dose of the radiation or the light during the first imaging operation by an arithmetic operation, and
the controller unit (106) controls the operation of the detector unit (101) based on the control signal so that the detector (101) performs the accumulation operation in the second imaging operation during the accumulation period.

2. The imaging system according to claim 1, wherein
the controlling computer (108) performs the arithmetic operation so as to suppress a step of image within a predetermined tolerable quantity or smaller, based on the information relating to the integral dose of the radiation or the light during the first imaging operation, and supplying the controller unit (106) with the control signal based on the accumulation period determined by the arithmetic operation, and
the controller unit (106) controls the operation of the detector unit (101) so that the detector (101) performs the accumulation operation in the second imaging operation during the accumulation period determined by the arithmetic operation.

3. The imaging system according to claim 2, wherein
the imaging system further comprises a radiation generator apparatus (110) irradiating the imaging apparatus (100) with the radiation,
the radiation generator apparatus (110) includes a mechanism having an irradiation function switchable between irradiations of the first and second irradiation fields, responsive to the control signal from the controlling computer (108), and
the controlling computer (108) performs the arithmetic operation, and supplies the controller unit (106) and the radiation generator apparatus (110) with the control signal based on the accumulation period determined by the arithmetic operation.

4. The imaging system according to claim 3, wherein
the controlling computer (108) includes a characteristic storage unit (504), a dosage detector unit (502), and an accumulation period determining unit (503),
the characteristic storage unit (504) stores data relating to the integral dose, the accumulation period and an output under a dark condition indicating characteristic of the detector unit (101), and stores an information relating to shortest irradiation period and a maximum output intensity of the radiation generator apparatus (110),
the dosage detector unit (502) outputs to the accumulation period determining unit (503) an information relating to the integral dose during the first imaging operation calculated based on an image data or data derived from a photo-timer provided separately from the detector (101), and
the accumulation period determining unit (503) determines the accumulation period based on an information relating to the integrating dosage and based on the data and the information stored in the characteristic storage unit (504).

5. The imaging system according to claim 4, wherein
the imaging system further comprises a control console (114) for outputting to the controlling computer (108) an information relating the dosage of the radiation necessary for the second imaging operation, and
the accumulation period determining unit (503) determines the accumulation period further based on the information relating the dosage of the radiation necessary for the second imaging operation.

6. The imaging system according to any one of claims 3 to 5, wherein
the arithmetic operation determines the accumulation period within a range of which lower limit is shortest irradiation period of the radiation generator apparatus (110).

7. The imaging system according to any one of claims 1 to 6, wherein
the pixel further includes a switch element (202) for outputting an electric signal based on the electric charge,
the detector (101) includes a detecting unit including the plurality of pixels arranged in the matrix, a driving circuit (102) for controlling a conducting state of the switch element (202) to drive the detecting unit and a read out circuit (103) for outputting, as an image data, the electric signal derived from the detecting unit through a signal wiring connected to the switch element (202),
the read out circuit (103) includes a reset switch for reset of the signal wiring, and
the controller unit (106) controls the driving circuit (102) and the reset switch such that the detector (101) performs an initializing operation for initializing the conversion element (202) during a period between the first and second imaging operations, responsive to the switching from the irradiation on the first irradiation fields (A) to the irradiation on the second irradiation fields (B).

8. The imaging system according to claim 7, wherein
the conversion element (201) is a MIS type conversion element,
the imaging system further comprises a power source unit (107) including a reference power source for supplying a reference voltage through the switch element (202) to one electrode of the conversion element (201), a refreshment power source for supplying a refreshment voltage through a refresh switch element to the one electrode of the conversion element (201), and a bias power source for supplying a bias voltage to the other electrode of the conversion element (201), and
the detector (101) performs a refreshment operation such that, under a condition of maintaining the switch element (202) at a non-conducting state and setting the refresh switch element at a conducting state, the other electrode is supplied with the bias voltage and the one electrode is supplied with the refreshment voltage through the refresh switch element, to refresh the conversion element (201).

9. An imaging apparatus (100) comprising:
a detector (101) including a plurality of pixels arranged in a matrix, wherein each of the pixels includes a conversion element (201) for converting a radiation or a light into an electric charge, such that the detector (101) performs an imaging operation for outputting an image data based on an irradiation with the radiation or the light, and
a controller unit (106) for controlling operations of the detector (101) including the imaging operation, wherein the imaging operation includes a first imaging operation for outputting an image data based on the irradiation with the light or the radiation on a first irradiation field (A) of the detector (101) corresponding to a part of the plurality of pixels, and a second imaging operation for outputting an image data based on the irradiation with the light or the radiation on a second irradiation field (B) of an area larger than that of the first irradiation field (A),
**characterized in that**
the controller unit (106) controls the operation of the detector unit (101) so that the detector (101) performs the accumulation operation in the second imaging operation during an accumulation period determined based on an information relating to an integral dose of the radiation or the light during the first imaging operation.

10. The imaging apparatus according to claim 9, wherein
the controller unit (106) controls the operation of the detector unit (101) so that the detector (101) performs the accumulation operation in the second imaging operation during the accumulation period determined by an arithmetic operation to suppress a step of image within a predetermined tolerable quantity or smaller, based on the information relating to an integral dose of the radiation or the light during the first imaging operation.

11. A controlling method of an imaging apparatus (100) comprising:
a detector (101) including a plurality of pixels arranged in a matrix, wherein each of the pixels includes a conversion element (201) for converting a radiation or a light into an electric charge, such that the detector (101) performs an imaging operation for outputting an image data based on an irradiation with the radiation or the light, wherein the controlling method controls operations of the detector (101) including the imaging operation and comprises:
a first imaging operation for outputting an image data based on the irradiation with the light or the radiation on a first irradiation field (A) of the detector (101) corresponding to a part of the plurality of pixels; and
a second imaging operation, conducted following to the first imaging operation, for outputting an image data based on the irradiation with the light or the radiation on a second irradiation field (B) of an area larger than that of the first irradiation field (A), during an accumulation period determined based on an information relating to an integral dose of the radiation or the light during the first imaging operation.

12. The controlling method according to claim 11, wherein
the second imaging operation is performed during the accumulation period determined by an arithmetic operation to suppress a step of image within a predetermined tolerable quantity or smaller, based on the information relating to the integral dose of the radiation or the light during the first imaging operation.

13. A program to set a computed to execute a controlling of an imaging apparatus (100) comprising:
a detector (101) including a plurality of pixels arranged in a matrix, wherein each of the pixels includes a conversion element (201) for converting a radiation or a light into an electric charge, such that the detector (101) performs an imaging operation for outputting an image data based on an irradiation with the radiation or the light, such that the computer controls operations of the detector (101) including the imaging operation, to perform:
a first imaging operation for outputting an image data based on the irradiation with the light or the radiation on a first irradiation field (A) of the detector (101) corresponding to a part of the plurality of pixels; and
a second imaging operation, conducted following to the first imaging operation, for outputting an image data based on the irradiation with the light or the radiation on a second irradiation field (B) of an area larger than that of the first irradiation field (A), during an accumulation period determined based on an information relating to an integral dose of the radiation or the light during the first imaging operation.

14. The program according to claim 13, wherein
the second imaging operation is performed during the accumulation period determined by an arithmetic operation to suppress a step of image within a predetermined tolerable quantity or smaller, based on the information relating to the integral dose of the radiation or the light during the first imaging operation.

## Patentansprüche

1. Bildgebungssystem mit
einer Bildgebungsvorrichtung (100), die eine Erfassungseinheit (101) mit einer Vielzahl von in einer Matrix angeordneten Bildelementen, wobei jedes der Bildelemente ein Umwandlungselement (201) zur Umwandlung einer Strahlung oder eines Lichts in eine elektrische Ladung derart enthält, dass die Erfassungseinheit (101) einen Bildgebungsbetrieb zur Ausgabe von Bilddaten basierend auf einer Bestrahlung mit der Strahlung oder dem Licht durchführt, und eine Steuereinheit (106) zur Steuerung von Betrieben der Erfassungseinheit (101) inklusive des Bildgebungsbetriebs umfasst, wobei der Bildgebungsbetrieb einen ersten Bildgebungsbetrieb zur Ausgabe von Bilddaten basierend auf der Bestrahlung mit dem Licht oder der Strahlung auf ein erstes Bestrahlungsfeld (A) der Erfassungseinheit (101) entsprechend einem Teil der Vielzahl von Bildelementen aufweist, und einen zweiten Bildgebungsbetrieb zur Ausgabe von Bilddaten basierend auf der Bestrahlung mit dem Licht oder der Strahlung auf ein zweites Bestrahlungsfeld (B) einer größeren Fläche als der des ersten Bestrahlungsfeldes (A) aufweist, und
einem Steuercomputer (108) zur Steuerung der Bildgebungsvorrichtung (100),
**dadurch gekennzeichnet, dass**
der Steuercomputer (108) die Steuereinheit (106) mit einem Steuersignal basierend auf einem Akkumulationszeitraum versorgt, der basierend auf einer Information bezüglich einer integralen Dosis der Strahlung oder des Lichts während dem ersten Bildgebungsbetrieb durch einen arithmetischen Betrieb bestimmt ist, und
die Steuereinheit (106) den Betrieb der Erfassungseinheit (101) basierend auf dem Steuersignal derart steuert, dass die Erfassungseinheit (101) den Akkumulationsbetrieb in dem zweiten Bildgebungsbetrieb während dem Akkumulationszeitraum durchführt.

2. Bildgebungssystem nach Anspruch 1, wobei
der Steuercomputer (108) den arithmetischen Betrieb durchführt, um eine Stufe eines Bildes auf innerhalb einer vorbestimmten tolerierbaren Menge oder kleiner basierend auf den Informationen bezüglich der integralen Dosis der Strahlung oder des Lichts während dem ersten Bildgebungsbetrieb zu unterdrücken, und die Steuereinheit (106) mit dem Steuersignal basierend auf dem durch den arithmetischen Betrieb bestimmten Akkumulationszeitraum versorgt, und
die Steuereinheit (106) den Betrieb der Erfassungseinheit (101) derart steuert, dass die Erfassungseinheit (101) den Akkumulationsbetrieb in dem zweiten Bildgebungsbetrieb während dem durch den arithmetischen Betrieb bestimmten Akkumulationszeitraum durchführt.

3. Bildgebungssystem nach Anspruch 2, wobei
das Bildgebungssystem ferner eine Strahlungserzeugungsvorrichtung (110) aufweist, die die Bildgebungsvorrichtung (100) mit der Strahlung bestrahlt,
die Strahlungserzeugungsvorrichtung (110) einen Mechanismus mit einer Bestrahlungsfunktion enthält, die zwischen Bestrahlungen der ersten und zweiten Bestrahlungsfelder in Abhängigkeit von dem Steuersignal von dem Steuercomputer (108) umschaltbar ist, und
der Steuercomputer (108) den arithmetischen Betrieb durchführt, und die Steuereinheit (106) und die Strahlungserzeugungsvorrichtung (110) mit dem Steuersignal basierend auf dem durch den arithmetischen Betrieb bestimmten Akkumulationszeitraum versorgt.

4. Bildgebungssystem nach Anspruch 3, wobei
der Steuercomputer (108) eine Eigenschaftsspeichereinheit (504), eine Dosiserfassungseinheit (502) und eine Akkumulationszeitraumbestimmungseinheit (503) umfasst,
die Eigenschaftsspeichereinheit (504) Daten bezüglich der integralen Dosis, des Akkumulationszeitraums, und einer auf eine Eigenschaft der Erfassungseinheit (101) hindeutende Ausgabe unter einer dunklen Bedingung speichert, und eine Information bezüglich eines kürzesten Bestrahlungszeitraums und einer maximalen Ausgabeintensität der Strahlungserzeugungsvorrichtung (110) speichert,
die Dosiserfassungseinheit (502) eine Information bezüglich der integralen Dosis während dem ersten Bildgebungsbetrieb an die Akkumulationszeitraumbestimmungseinheit (503) ausgibt, die basierend auf Bilddaten oder von einem separat von der Erfassungseinheit (101) bereitgestellten Fotozeitmesser erlangten Daten berechnet ist, und
die Akkumulationszeitraumbestimmungseinheit (503) den Akkumulationszeitraum basierend auf einer Information bezüglich der integrierenden Dosis und basierend auf den in der Eigenschaftsspeichereinheit (504) gespeicherten Daten und Informationen bestimmt.

5. Bildgebungssystem nach Anspruch 4, wobei
das Bildgebungssystem ferner eine Steuerkonsole (114) zur Ausgabe einer Information bezüglich der Dosis der für den zweiten Bildgebungsbetrieb notwendigen Strahlung an den Steuercomputer (108) umfasst, und
die Akkumulationszeitraumbestimmungseinheit (503) den Akkumulationszeitraum ferner basierend auf der Information bezüglich der Dosis der für den zweiten Bildgebungsbetrieb notwendigen Strahlung bestimmt.

6. Bildgebungssystem nach einem der Ansprüche 3 bis 5, wobei
der arithmetische Betrieb den Akkumulationszeitraum innerhalb einem Bereich bestimmt, dessen untere Grenze einen kürzesten Bestrahlungszeitraum der Strahlungserzeugungsvorrichtung (110) ist.

7. Bildgebungssystem nach einem der Ansprüche 1 bis 6, wobei
die Bildelemente ferner ein Schaltelement (202) zur Ausgabe eines elektrischen Signals basierend auf der elektrischen Ladung aufweisen,
die Erfassungseinheit (101) eine erfassende Einheit, die die Vielzahl von in der Matrix angeordneten Bildelementen enthält, einen Ansteuerschaltkreis (102) zur Steuerung eines leitenden Zustands des Schaltelements (202), um die erfassende Einheit anzusteuern, und einen Ausleseschaltkreis (103) aufweist zur Ausgabe des von der erfassenden Einheit erlangten elektrischen Signals als Bilddaten durch eine mit dem Schaltelement (202) verbundene Signalverkabelung,
der Ausleseschaltkreis (103) einen Rücksetzschalter zum Rücksetzen der Signalverkabelung enthält, und
die Steuereinheit (106) den Ansteuerschaltkreis (102) und den Rücksetzschalter derart steuert, dass die Erfassungseinheit (101) einen Vorbereitungsbetrieb zur Vorbereitung des Umwandlungselements (202) während einem Zeitraum zwischen den ersten und zweiten Bildgebungbetrieben durchführt in Abhängigkeit von dem Umschalten von der Bestrahlung auf die ersten Bestrahlungsfelder (A) zu der Bestrahlung auf die zweiten Bestrahlungsfelder (B).

8. Bildgebungssystem nach Anspruch 7, wobei
das Umwandlungselement (201) ein Umwandlungselement vom MIS-Typ ist,
das Bildgebungssystem ferner eine Energiequelleneinheit (107) aufweist mit einer Referenzenergiequelle zur Zufuhr einer Referenzspannung durch das Schaltelement (202) zu einer Elektrode des Umwandlungselements (201), einer Auffrischungsenergiequelle zur Zufuhr einer Auffrischungsspannung durch ein Auffrischschaltelement zu der einen Elektrode des Umwandlungselements (201), und einer Vorspannungsenergiequelle zur Zufuhr einer Vorspannungsspannung zu der anderen Elektrode des Umwandlungselements (201), und
die Erfassungseinheit (101) einen Auffrischungsbetrieb derart durchführt, dass unter einer Bedingung eines Aufrechterhaltens des Schaltelements (202) in einem nichtleitenden Zustand und eines Einstellens des Auffrischschaltelements in einen leitenden Zustand die andere Elektrode mit der Vorspannungsspannung versorgt ist und die eine Elektrode mit der Auffrischungsspannung durch das Auffrischschaltelement versorgt ist, um das Umwandlungselement (201) aufzufrischen.

9. Bildgebungsvorrichtung (100), mit
einer Erfassungseinheit (101) mit einer Vielzahl von in einer Matrix angeordneten Bildelementen, wobei jedes der Bildelemente ein Umwandlungselement (201) zur Umwandlung einer Strahlung oder eines Lichts in eine elektrische Ladung derart umfasst, dass die Erfassungseinheit (101) einen Bildgebungsbetrieb zur Ausgabe von Bilddaten basierend auf einer Bestrahlung mit der Strahlung oder dem Licht durchführt, und
einer Steuereinheit (106) zur Steuerung von Betrieben der Erfassungseinheit (101) inklusive des Bildgebungsbetriebs, wobei der Bildgebungsbetrieb einen ersten Bildgebungsbetrieb zur Ausgabe von Bilddaten basierend auf der Bestrahlung mit dem Licht oder der Strahlung auf ein erstes Bestrahlungsfeld (A) der Erfassungseinheit (101) entsprechend einem Teil der Vielzahl von Bildelementen und einen zweiten Bildgebungsbetrieb zur Ausgabe von Bilddaten basierend auf der Bestrahlung mit dem Licht oder der Strahlung auf ein zweites Bestrahlungsfeld (B) einer Fläche größer als der des ersten Bestrahlungsfelds (A) umfasst,
**dadurch gekennzeichnet, dass**
die Steuereinheit (106) den Betrieb der Erfassungseinheit (101) derart steuert, dass die Erfassungseinheit (101) den Akkumulationsbetrieb in dem zweiten Bildgebungsbetrieb während einem Akkumulationszeitraum durchführt, die basierend auf einer Information bezüglich einer integralen Dosis der Strahlung oder des Lichts während dem ersten Bildgebungsbetrieb bestimmt ist.

10. Bildgebungsvorrichtung nach Anspruch 9, wobei
die Steuereinheit (106) den Betrieb der Erfassungseinheit (101) derart steuert, dass die Erfassungseinheit (101) den Akkumulationsbetrieb in dem zweiten Bildgebungsbetrieb während dem durch einen arithmetischen Betrieb bestimmten Akkumulationszeitraum durchführt, um eine Stufe eines Bildes auf innerhalb einer vorbestimmten tolerierbaren Menge oder kleiner zu unterdrücken basierend auf der Information bezüglich einer integralen Dosis der Strahlung oder des Lichts während dem ersten Bildgebungsbetrieb.

11. Steuerverfahren einer Bildgebungsvorrichtung (100), mit
einer Erfassungseinheit (101) mit einer Vielzahl von in einer Matrix angeordneten Bildelementen, wobei jedes der Bildelemente ein Umwandlungselement (201) zur Umwandlung einer Strahlung oder eines Lichts in eine elektrische Ladung derart aufweist, dass die Erfassungseinheit (101) einen Bildgebungstrieb zur Ausgabe von Bilddaten basierend auf einer Bestrahlung mit der Strahlung oder dem Licht durchführt, wobei das Steuerverfahren Betriebe der Erfassungseinheit (101) inklusive des Bildgebungsbetriebs steuert und aufweist
einen ersten Bildgebungsbetrieb zum Ausgeben von Bilddaten basierend auf der Bestrahlung mit dem Licht oder der Strahlung auf ein erstes Bestrahlungsfeld (A) der Erfassungseinheit (101) entsprechend einem Teil der Vielzahl von Bildelementen, und
einen zweiten Bildgebungsbetrieb, der auf den ersten Bildgebungsbetrieb hin eingeleitet wird, zum Ausgeben von Bilddaten basierend auf der Bestrahlung mit dem Licht oder der Strahlung auf ein zweites Bestrahlungsfeld (B) einer Fläche größer als der des ersten Bestrahlungsfelds (A) während einem Akkumulationszeitraum, die basierend auf einer Information bezüglich einer integralen Dosis der Strahlung oder des Lichts während dem ersten Bildgebungsbetrieb bestimmt ist.

12. Steuerverfahren nach Anspruch 11, wobei
der zweite Bildgebungsbetrieb während dem durch einen arithmetischen Betrieb bestimmten Akkumulationszeitraum durchgeführt wird, um eine Stufe eines Bildes auf innerhalb einer vorbestimmten tolerierbaren Menge oder kleiner zu unterdrücken basierend auf der Information bezüglich der integralen Dosis der Strahlung oder des Lichts während dem ersten Bildgebungsbetrieb.

13. Programm, um einen Computer einzustellen, um eine Steuerung einer Bildgebungsvorrichtung (100) mit
einer Erfassungseinheit (101) mit einer Vielzahl von in einer Matrix angeordneten Bildelementen, wobei jedes der Bildelemente ein Umwandlungselement (201), zur Umwandlung einer Strahlung oder eines Lichts in eine elektrische Ladung derart aufweist, dass die Erfassungseinheit (101) einen Bildgebungstrieb zur Ausgabe von Bilddaten basierend auf einer Bestrahlung mit der Strahlung oder dem Licht durchführt, derart auszuführen, dass der Computer Betriebe der Erfassungseinheit (101) inklusive des Bildgebungsbetriebs steuert, um durchzuführen
einen ersten Bildgebungsbetrieb zum Ausgeben von Bilddaten basierend auf der Bestrahlung mit dem Licht oder der Strahlung auf ein erstes Bestrahlungsfeld (A) der Erfassungseinheit (101) entsprechend einem Teil der Vielzahl von Bildelementen, und
einen zweiten Bildgebungsbetrieb, der auf den ersten Bildgebungsbetrieb hin eingeleitet wird, zum Ausgeben von Bilddaten basierend auf der Bestrahlung mit dem Licht oder der Strahlung auf ein zweites Bestrahlungsfeld (B) einer Fläche größer als der des ersten Bestrahlungsfelds (A) während einem Akkumulationszeitraum, der basierend auf einer Information bezüglich einer integralen Dosis der Strahlung oder des Lichts während dem ersten Bildgebungsbetrieb bestimmt wird.

14. Programm nach Anspruch 13, wobei
der zweite Bildgebungsbetrieb während dem durch einen arithmetischen Betrieb bestimmten Akkumulationszeitraum durchgeführt wird, um eine Stufe eines Bildes auf innerhalb einer vorbestimmten tolerierbaren Menge oder kleiner zu unterdrücken basierend auf der Information bezüglich der integralen Dosis der Strahlung oder des Lichts während dem ersten Bildgebungsbetrieb.

## Revendications

1. Système d'imagerie comprenant :
un équipement (100) de formation d'image comprenant un détecteur (101) incluant une pluralité de pixels agencés en une matrice, chacun des pixels incluant un élément (201) de conversion destiné à convertir un rayonnement ou de la lumière en une charge électrique, de sorte que le détecteur (101) effectue une opération de formation d'image pour sortir une donnée d'image en se basant sur une irradiation par le rayonnement ou la lumière, et une unité (106) de commande destinée à commander le fonctionnement du détecteur (101) y compris l'opération de formation d'image, dans lequel l'opération de formation d'image inclut une première opération de formation d'image destinée à sortir une donnée d'image en se basant sur l'irradiation par la lumière ou le rayonnement sur une première zone (A) d'irradiation du détecteur (101) correspondant à une partie de la pluralité de pixels, et une seconde opération de formation d'image destinée à sortir une donnée d'image en se basant sur l'irradiation par la lumière ou le rayonnement sur une seconde zone (B) d'irradiation d'une superficie plus grande que celle de la première zone (A) d'irradiation ; et
un processeur (108) de commande destiné à commander l'équipement (100) de formation d'image,
**caractérisé :**
**en ce que** le processeur (108) de commande alimente l'unité (106) de commande avec un signal de commande basé sur une période d'accumulation déterminée, par une opération arithmétique, en se basant sur une information relative à une dose intégrale du rayonnement ou de la lumière durant la première opération de formation d'image ; et
**en ce que** l'unité (106) de commande commande le fonctionnement de l'unité (101) de détection, en se basant sur le signal de commande, de façon que le détecteur (101) effectue l'opération d'accumulation dans la seconde opération de formation d'image durant la période d'accumulation.

2. Système d'imagerie selon la revendication 1,
dans lequel le processeur (108) de commande effectue l'opération arithmétique de façon à limiter un effet d'échelons de l'image au maximum à une valeur admissible prédéterminée, en se basant sur l'information relative à la dose intégrale du rayonnement ou de la lumière durant la première opération de formation d'image, et en alimentant l'unité (106) de commande avec le signal de commande basé sur la période d'accumulation déterminée par l'opération arithmétique, et
dans lequel l'unité (106) de commande commande le fonctionnement de l'unité (101) de détection de façon que le détecteur (101) effectue l'opération d'accumulation dans la seconde opération de formation d'image durant la période d'accumulation déterminée par l'opération arithmétique.

3. Système d'imagerie selon la revendication 2,
dans lequel le système d'imagerie comprend en outre un équipement (110) générateur de rayonnement irradiant l'équipement (100) de formation d'image à l'aide du rayonnement,
dans lequel l'équipement (110) générateur de rayonnement inclut un mécanisme ayant une fonction d'irradiation commutable entre les irradiations des première et seconde zones d'irradiation, en réponse à un signal de commande provenant du processeur (108) de commande, et
dans lequel le processeur (108) de commande effectue l'opération arithmétique, et alimente l'unité (106) de commande et l'équipement (110) générateur de rayonnement avec le signal de commande basé sur la période d'accumulation déterminée par l'opération arithmétique.

4. Système d'imagerie selon la revendication 3,
dans lequel le processeur (108) de commande inclut une unité (504) de mémorisation de caractéristique, une unité (502) de détection de dosage, et une unité (503) de détermination de période d'accumulation,
dans lequel l'unité (504) de mémorisation de caractéristique mémorise des données relatives à la dose intégrale, à la période d'accumulation et à une sortie à l'état noir représentant la caractéristique de l'unité (101) de détection, et mémorise une information relative à la période d'irradiation la plus courte et à une intensité maximale de sortie de l'équipement (110) générateur de rayonnement,
dans lequel l'unité (502) de détection de dosage sort vers l'unité (503) de détermination de période d'accumulation une information relative à la dose intégrale durant la première opération de formation d'image calculée en se basant sur une donnée d'image ou sur une donnée obtenue à partir d'un compte-pose prévu séparément du détecteur (101), et
dans lequel l'unité (503) de détermination de période d'accumulation détermine la période d'accumulation en se basant sur une information relative au dosage d'intégration et en se basant sur la donnée et l'information mémorisée dans l'unité (504) de mémorisation de caractéristique.

5. Système d'imagerie selon la revendication 4,
dans lequel le système d'imagerie comprend en outre un pupitre (114) de commande destiné à sortir vers le processeur (108) de commande une information relative au dosage du rayonnement nécessaire pour la seconde opération de formation d'image, et
dans lequel l'unité (503) de détermination de période d'accumulation détermine la période d'accumulation en se basant en outre sur l'information relative au dosage du rayonnement nécessaire pour la seconde opération de formation d'image.

6. Système d'imagerie selon l'une quelconque des revendications 3 à 5, dans lequel l'opération arithmétique détermine la période d'accumulation à l'intérieur d'une plage dont la limite inférieure est la période de rayonnement la plus courte de l'équipement (110) générateur de rayonnement.

7. Système d'imagerie selon l'une quelconque des revendications 1 à 6,
dans lequel le pixel inclut en outre un élément (202) de commutation destiné à sortir un signal électrique basé sur la charge électrique,
dans lequel le détecteur (101) inclut une unité de détection incluant la pluralité de pixels agencés en matrice, un circuit (102) d'attaque destiné à commander un état conducteur de l'élément (202) de commutation pour attaquer l'unité de détection et un circuit (103) de lecture destiné à sortir, en tant que donnée d'image, le signal électrique obtenu de l'unité de détection par l'intermédiaire d'un câblage de signal connecté à l'élément (202) de commutation,
dans lequel le circuit (103) de lecture inclut un commutateur de réinitialisation destiné à réinitialiser le câblage de signal, et
dans lequel l'unité (106) de commande commande le circuit (102) d'attaque et le commutateur de réinitialisation de sorte que le détecteur (101) effectue une opération d'initialisation pour initialiser l'élément (202) de conversion durant une période entre les première et seconde opérations de formation d'image, en réponse à la commutation de l'irradiation sur les premières zones (A) d'irradiation en irradiation sur les secondes zones (B) d'irradiation.

8. Système d'imagerie selon la revendication 7,
dans lequel l'élément (201) de conversion est un élément de conversion du type MIS (pour "Métal-Isolant-Semi-conducteur"),
dans lequel le système d'imagerie comprend en outre une unité (107) d'alimentation incluant une alimentation de référence destinée à délivrer une tension de référence par l'intermédiaire de l'élément (202) de commutation à une première électrode de l'élément (201) de conversion, et une alimentation de rafraîchissement destinée à délivrer une tension de rafraîchissement par l'intermédiaire d'un élément de commutation de rafraîchissement à la première électrode de l'élément (201) de conversion, et une alimentation de polarisation destinée à délivrer une tension de polarisation à l'autre électrode de l'élément (201) de conversion, et
dans lequel le détecteur (101) effectue une opération de rafraîchissement de sorte que, sous condition du maintien de l'élément (202) de commutation à un état non conducteur et de la mise de l'élément de commutation de rafraîchissement à un état conducteur, l'autre électrode est alimentée avec la tension de polarisation et la première électrode est alimentée avec la tension de rafraîchissement par l'intermédiaire de l'élément de commutation de rafraîchissement, pour rafraîchir l'élément (201) de conversion.

9. Appareil d'imagerie (100) comprenant :
un détecteur (101) incluant une pluralité de pixels agencés en une matrice, chacun des pixels incluant un élément (201) de conversion destiné à convertir un rayonnement ou de la lumière en une charge électrique, de sorte que le détecteur (101) effectue une opération de formation d'image pour sortir une donnée d'image en se basant sur une irradiation par le rayonnement ou la lumière, et
une unité (106) de commande destinée à commander le fonctionnement du détecteur (101) y compris l'opération de formation d'image, dans lequel l'opération de formation d'image inclut une première opération de formation d'image destinée à sortir une donnée d'image en se basant sur l'irradiation par la lumière ou le rayonnement sur une première zone (A) d'irradiation du détecteur (101) correspondant à une partie de la pluralité de pixels, et une seconde opération de formation d'image destinée à sortir une donnée d'image en se basant sur l'irradiation par la lumière ou le rayonnement sur une seconde zone (B) d'irradiation d'une superficie plus grande que celle de la première zone (A) d'irradiation ; et
**caractérisé :**
**en ce que** l'unité (106) de commande commande le fonctionnement de l'unité (101) de détection de façon que le détecteur (101) effectue l'opération d'accumulation dans la seconde opération de formation d'image durant une période d'accumulation déterminée en se basant sur une information relative à une dose intégrale du rayonnement ou de la lumière durant la première opération de formation d'image.

10. Appareil d'imagerie selon la revendication 9, dans lequel l'unité (106) de commande commande le fonctionnement de l'unité (101) de détection de façon que le détecteur (101) effectue l'opération d'accumulation dans la seconde opération de formation d'image durant la période d'accumulation déterminée par une opération arithmétique de façon à limiter un effet d'échelons de l'image au maximum à une valeur admissible prédéterminée, en se basant sur l'information relative à une dose intégrale du rayonnement ou de la lumière durant la première opération de formation d'image.

11. Procédé de commande d'un appareil d'imagerie (100) comprenant :
un détecteur (101) incluant une pluralité de pixels agencés en une matrice, chacun des pixels incluant un élément (201) de conversion destiné à convertir un rayonnement ou de la lumière en une charge électrique, de sorte que le détecteur (101) effectue une opération de formation d'image pour sortir une donnée d'image en se basant sur une irradiation par le rayonnement ou la lumière, dans lequel le procédé de commande commande le fonctionnement du détecteur (101) y compris l'opération de formation d'image et comprend :
une première opération de formation d'image destinée à sortir une donnée d'image en se basant sur l'irradiation par la lumière ou le rayonnement sur une première zone (A) d'irradiation du détecteur (101) correspondant à une partie de la pluralité de pixels ; et
une seconde opération de formation d'image, menée à la suite de la première opération de formation d'image, destinée à sortir une donnée d'image en se basant sur l'irradiation par la lumière ou le rayonnement sur une seconde zone (B) d'irradiation d'une superficie plus grande que celle de la première zone (A) d'irradiation, durant une période d'accumulation déterminée en se basant sur une information relative à une dose intégrale du rayonnement ou de la lumière durant la première opération de formation d'image.

12. Procédé de commande selon la revendication 11, dans lequel la seconde opération de formation d'image est effectuée durant la période d'accumulation déterminée par une opération arithmétique de façon à limiter un effet d'échelons de l'image au maximum à une valeur admissible prédéterminée, en se basant sur l'information relative à la dose intégrale du rayonnement ou de la lumière durant la première opération de formation d'image.

13. Programme pour faire qu'un processeur effectue une commande d'un appareil d'imagerie (100) comprenant :
un détecteur (101) incluant une pluralité de pixels agencés en une matrice, chacun des pixels incluant un élément (201) de conversion destiné à convertir un rayonnement ou de la lumière en une charge électrique, de sorte que le détecteur (101) effectue une opération de formation d'image pour sortir une donnée d'image en se basant sur une irradiation par le rayonnement ou la lumière, de sorte que le processeur commande le fonctionnement du détecteur (101) y compris l'opération de formation d'image, pour effectuer :
une première opération de formation d'image destinée à sortir une donnée d'image en se basant sur l'irradiation par la lumière ou le rayonnement sur une première zone (A) d'irradiation du détecteur (101) correspondant à une partie de la pluralité de pixels ; et
une seconde opération de formation d'image, menée à la suite de la première opération de formation d'image, destinée à sortir une donnée d'image en se basant sur l'irradiation par la lumière ou le rayonnement sur une seconde zone (B) d'irradiation d'une superficie plus grande que celle de la première zone (A) d'irradiation, durant une période d'accumulation déterminée en se basant sur une information relative à une dose intégrale du rayonnement ou de la lumière durant la première opération de formation d'image.

14. Programme selon la revendication 13, dans lequel la seconde opération de formation d'image est effectuée durant la période d'accumulation déterminée par une opération arithmétique de façon à limiter un effet d'échelons de l'image au maximum à une valeur admissible prédéterminée, en se basant sur l'information relative à la dose intégrale du rayonnement ou de la lumière durant la première opération de formation d'image.
